# EUROPEAN PATENT APPLICATION

(11) **EP 2 484 366 A1**
(43) Date of publication of application: **08.08.2012**
(21) Application number: 11305119.7
(22) Date of filing: 07.02.2011
(51) Int. Cl.: A61K 31/7056, A61K 31/715, A61P 7/02

(54) **Idrabiotaparinux for the treatment of pulmonary embolism and for the secondary prevention of venous thromboembolic events**

(71) Applicant: SANOFI, 75008 Paris (FR)
(72) Inventor: The designation of the inventor has not yet been filed
(74) Representative: Romanowski, Caroline

(57) **Abstract**

The invention relates to idrabiotaparinux for use in the treatment of pulmonary embolism in patients with or without deep venous thrombosis and for the secondary prevention of venous thromboembolic events in said patients, wherein the efficacy and safety of said uses are clinically proven by a phase III clinical trial.

## Description

The invention relates to the use of idrabiotaparinux in the treatment of pulmonary embolism in patients with or without deep venous thrombosis and for the secondary prevention of venous thromboembolic events in said patients.

Idrabiotaparinux (International Non-proprietary Name), or SSR126517 (laboratory code), is developed by sanofi-aventis as the first long-acting anticoagulant administered once-weekly by subcutaneous route, with the unique property to be almost instantly and specifically neutralizable by intravenous administration of avidin. It is developed as an alternative to vitamin K antagonists (VKA).

Idrabiotaparinux is the biotinylated pentasaccharide corresponding to the structure depicted below.

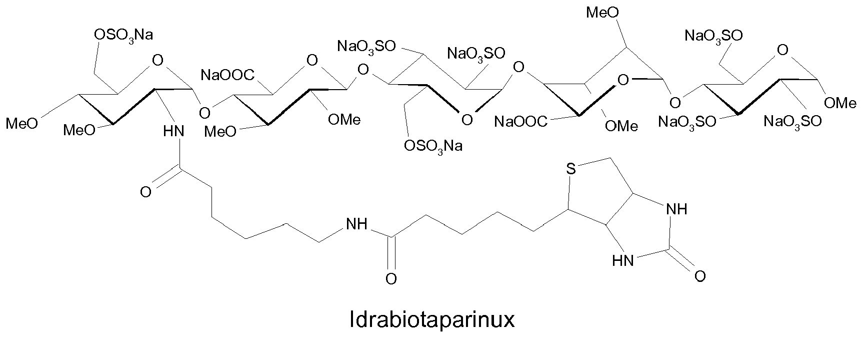

The pentasaccharide structure of idrabiotaparinux is the same as idraparinux, another antithrombotic agent developed by sanofi-aventis (see structure below). However in idrabiotaparinux, the presence of a biotin hook covalently linked to the first saccharidic unit enables the compound to be neutralized by avidin or streptavidin, as described in the international patent application WO 02/24754.

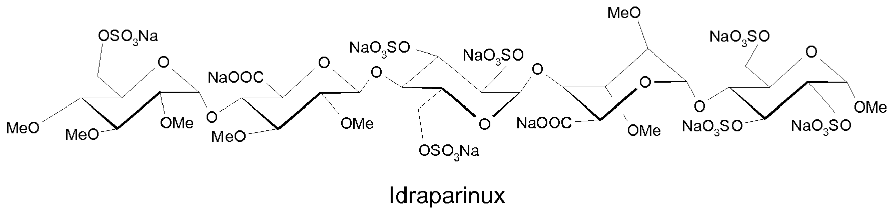

In the EQUINOX trial, which enrolled 757 patients with DVT treated for 6 months with equimolar doses of either idrabiotaparinux or idraparinux, idrabiotaparinux was demonstrated to be bioequipotent to idraparinux in terms of pharmacokinetics and pharmacodynamics, in patients with symptomatic deep venous thrombosis (Journal of Thrombosis and Haemostasis, 2010, Vol. 9, p. 92-99). The results of this bioequipotency trial indicated that idrabiotaparinux could be a suitable treatment for patients with deep venous thrombosis. However, the apparent failure of idraparinux in patients with pulmonary embolism indicated the need for a formal evaluation of idrabiotaparinux in this patient group (N. Eng. J. Med., 2007, Vol. 357, p. 1094-104).

It has now been demonstrated, in a phase III study involving 3202 patients with pulmonary embolism, that idrabiotaparinux is a safe and effective drug in the treatment of pulmonary embolism in patients with or without deep venous thrombosis and in the secondary prevention of venous thromboembolic events in said patients.

The invention therefore relates to idrabiotaparinux for use in the treatment of pulmonary embolism in patients with or without deep venous thrombosis and for the secondary prevention of venous thromboembolic events in said patients, wherein the efficacy and safety of said uses are clinically proven by a phase III clinical trial.

According to the instant invention, the terms below have the following meanings:
- "idrabiotaparinux" designates the sodium salt of this compound, as defined above, or any other pharmaceutically acceptable salt thereof;
- a "phase III clinical trial" refers to an international, multicenter, randomized, double-blind, double-dummy, parallel group study involving a large patients group (3202 patients in the instant invention), aiming at being the definitive assessment of how effective and safe the drug is, in comparison with current standard treatment;
- "deep venous thrombosis" refers to a blood clot in a deep vein of the lower limbs;
- a "patient" refers to a patient with confirmed acute symptomatic pulmonary embolism or who has previously manifested symptoms of pulmonary embolism, with or without symptomatic deep venous thrombosis of the lower limbs;
- "treatment" refers to the administration of a therapy to an individual who already manifests at least one symptom of a disease or condition (in the instant case, pulmonary embolism) or who has previously manifested at least one symptom of such a disease or condition. The term "treatment" in the framework of the instant invention therefore encompasses both a curative treatment and a treatment for preventing recurrences of pulmonary embolism;
- "secondary prevention" refers to a treatment for the prevention of recurrences of thromboembolic events, including pulmonary embolism and deep venous thrombosis.

As used herein, the wording "idrabiotaparinux for use in ..." shall be understood as being equivalent to the wording "use of idrabiotaparinux for ..." or "use of idrabiotaparinux for the preparation of a medicament for use in ...".

In the instant invention, the phase III clinical trial enrolled 3202 patients. The patients included in the clinical trial had confirmed acute symptomatic pulmonary embolism, with or without deep venous thrombosis of the lower limbs, as it will be described in details hereafter.

In an embodiment of the invention, idrabiotaparinux is administered for 3 months.

In another embodiment of the invention, idrabiotaparinux is administered for 6 months.

In another embodiment of the invention, idrabiotaparinux is administered at a 3.0 mg dose once weekly for patients without severe renal insufficiency (creatinine clearance ≥ 30 mL/min).

In another embodiment of the invention, idrabiotaparinux is administered at a 1.8 mg dose once weekly for patients with severe renal insufficiency (creatinine clearance < 30 mL/min).

According to the instant invention, the efficacy and safety of idrabiotaparinux for the treatment of pulmonary embolism in patients with or without deep venous thrombosis and for the secondary prevention of venous thromboembolic events in said patients are assessed compared to a vitamin K antagonist, namely warfarin, as standard antithrombotic treatment.

According to the instant invention, said venous thromboembolic events are selected from pulmonary embolism (fatal or not) and deep venous thrombosis.

In another embodiment of the invention, idrabiotaparinux displays an improved safety in terms of bleedings (defined as any clinically relevant bleedings), including major bleedings, compared to a vitamin K antagonist as standard antithrombotic treatment (namely, warfarin). This effect is more particularly observed 3 months after start of the treatment with idrabiotaparinux.

According to the instant invention, the term "bleedings" designates any clinically relevant bleeding (i.e. major or clinically relevant non-major hemorrhage).

According to the instant invention, the term "major bleedings" designates any of the following clinical situations:
- bleeding associated with a fall in hemoglobin of 2 g per deciliter or more,
- bleeding that led to a transfusion of 2 or more units of packed red cells or whole blood (a red cell unit being defined as the quantity of red cells obtained from or corresponding to approximately 500 ml of whole blood),
- bleeding that involved a critical organ (intracranial, intraocular, intraspinal, retroperitoneal or pericardial),
- bleeding that contributed to death.

According to the instant invention, the term "clinically relevant non-major bleeding" designates any of the following clinical situations:
- bleeding compromising hemodynamics,
- bleeding leading to hospitalization,
- subcutaneous hematoma larger than 25 cm², or 100 cm² if there was a traumatic cause,
- intramuscular hematoma documented by ultrasonography,
- epistaxis that lasted for more than 5 minutes, was repetitive (i.e., two or more episodes of bleeding more extensive than spots on a handkerchief within 24 hours), or led to an intervention (e.g., packing or electrocoagulation),
- gingival bleeding occurring spontaneously (i.e., unrelated to eating or tooth brushing) or lasting for more than 5 minutes,
- hematuria that was macroscopic and was spontaneous or lasted for more than 24 hours after instrumentation (e.g., catheter placement or surgery) of the urogenital tract,
- macroscopic gastrointestinal hemorrhage, including at least one episode of melena or hematemesis, if clinically apparent with positive results on a fecal occult-blood test,
- rectal blood loss, if more than a few spots on toilet paper,
- hemoptysis, if more than a few speckles in the sputum and not occurring within the context of pulmonary embolism,
- other bleeding type considered to have clinical consequences for a patient:
   - such as medical intervention, the need for unscheduled contact (visit or telephone call) with a physician, or temporary cessation of a study drug,
   - or associated with pain or impairment of activities of daily life.

As described above, idrabiotaparinux has demonstrated its efficacy and its improved safety in the treatment of pulmonary embolism in patients with or without deep venous thrombosis and for the secondary prevention of venous thromboembolic events in said patients. As a consequence, another embodiment of the invention is the improved benefit-risk ratio of idrabiotaparinux compared to a vitamin K antagonist as standard antithrombotic treatment (namely, warfarin).

Said improved benefit-risk ratio is present after 3 months and after 6 months of treatment with idrabiotaparinux, administered once weekly as described above.

In addition, idrabiotaparinux enables a long term protection of the patients to whom it has been administered.

The invention therefore also relates to idrabiotaparinux for use in the treatment of pulmonary embolism in patients with or without deep venous thrombosis and for the secondary prevention of venous thromboembolic events in said patients, wherein the efficacy of idrabiotaparinux in said uses and its improved benefit-risk ratio remain present after treatment discontinuation, up to 12 months after start of the treatment with idrabiotaparinux.

More particularly, the invention relates to idrabiotaparinux for use in the extended prevention of venous thromboembolic events for an additional 6-month period after discontinuation of an initial 6-month treatment with idrabiotaparinux, in patients with or without deep venous thrombosis.

In the framework of the instant invention, the term "extended prevention" designates a protective effect against thromboembolic events (i.e. pulmonary embolism and deep venous thrombosis) in a period following discontinuation of the treatment with idrabiotaparinux, i.e. a prolonged protective effect. The term "discontinuation" designates a permanent interruption of the idrabiotaparinux treatment by the patient.

Hence, the invention also relates to idrabiotaparinux for use in the prevention of recurrence of thromboembolic events, in patients with or without deep venous thrombosis, for 6 months after discontinuation of an initial 6-month treatment with idrabiotaparinux.

The invention also relates to an article of manufacture comprising:
- a packaging material,
- a compound chosen from idrabiotaparinux or a pharmaceutically acceptable salt thereof, and
- a label or package insert contained within said packaging material indicating that said compound is effective as an antithrombotic treatment for the treatment of pulmonary embolism in patients with or without deep venous thrombosis and for the secondary prevention of venous thromboembolic events in said patients.

The invention also relates to an article of manufacture comprising:
- a packaging material,
- a compound chosen from idrabiotaparinux or a pharmaceutically acceptable salt thereof, and
- a label or package insert contained within said packaging material indicating that said compound is safe as an antithrombotic treatment for the treatment of pulmonary embolism in patients with or without deep venous thrombosis and for the secondary prevention of venous thromboembolic events in said patients.

In another embodiment, the invention relates to a pharmaceutical composition comprising idrabiotaparinux, useful for the treatment of pulmonary embolism in patients with or without deep venous thrombosis and for the secondary prevention of venous thromboembolic events in said patients. Such a pharmaceutical composition advantageously comprises idrabiotaparinux, at a dose of 3.0 mg or 1.8 mg for example, as well as pharmaceutically acceptable and inert excipients. Such excipients are chosen among those known in the Art, according to the desired pharmaceutical formulation and mode of administration. An advantageous pharmaceutical composition according to the invention is an injectable formulation adapted to the subcutaneous route.

In another embodiment, the invention relates to the use of idrabiotaparinux for the manufacture of a medicament useful in the treatment of pulmonary embolism in patients with or without deep venous thrombosis and useful in the secondary prevention of venous thromboembolic events in said patients, wherein the efficacy and safety of said use is clinically proven by a phase III clinical trial.

The invention will be more clearly understood by reference to the following examples of the invention, which are included herewith for purposes of illustration only and are not intended to be limiting the invention.

The following abbreviations will be used:
- ALAT :: alanine aminotransferase
- aPTT :: activated partial thromboplastin time
- ASAT :: aspartate aminotransferase
- AT :: antithrombin
- b.i.d. :: *bis in die* (twice daily)
- CI :: confidence interval
- CIAC:: central and independent adjudication committee
- CT :: computed tomography
- CUS :: compression ultrasonography
- DVT:: deep venous thrombosis
- INR:: International Normalized Ratio
- i.v. :: intravenous
- IVRS :: Interactive Voice Response System
- LMWH:: Low Molecular Weight Heparin
- N :: number of patients
- PE:: pulmonary embolism
- PK :: pharmacokinetics
- s.c. :: subcutaneous
- SRI :: severe renal insufficiency
- SSR126517E :: idrabiotaparinux, in the form of its sodium salt
- SSR29261 :: avidin
- UFH :: Unfractionated Heparin
- ULN :: upper limit of normal range
- VKA :: vitamin K antagonist
- VPLS:: Ventilation/Perfusion Lung Scintigraphy
- VTE :: venous thrombo-embolic events

### Description of the figures:

- Figure 1 represents the Kaplan-Meier cumulative incidence of PE/DVT (fatal or not) in the combined 3-month and 6-month period (randomized population).
- Figure 2 represents the Kaplan-Meier cumulative incidence of PE/DVT (fatal or not) up to the end of study (randomized population).
- Figure 3 represents the Kaplan-Meier cumulative incidence of clinically relevant bleeding in the combined 3-month and 6-month period (randomized population).
- Figure 4 represents the Kaplan-Meier cumulative incidence of clinically relevant bleeding up to the end of study (randomized population).

### CASSIOPEA (EFC6034) clinical trial

An international, multicenter, randomized, double-blind, double-dummy, parallel group, study of 3-month or 6-month treatment with SSR126517E (3.0 mg subcutaneous route once weekly) versus oral INR-adjusted warfarin in the treatment of patients with symptomatic pulmonary embolism with or without symptomatic deep venous thrombosis.

### 1) Objectives

Primary efficacy objective : to evaluate whether a 3- or 6-month treatment with once-weekly SSR126517E s.c. injections is at least as effective as a 3- or 6-month treatment with INR-adjusted warfarin in the treatment and prevention of venous thrombo-embolic events (VTE) recurrence at 3 months in patients with symptomatic pulmonary embolism (PE) with or without symptomatic deep venous thrombosis (DVT).

Main secondary efficacy objective : to evaluate whether a 6-month treatment with once-weekly SSR126517E s.c. injections is at least as effective as a 6-month treatment with INR-adjusted warfarin in the treatment and prevention of VTE recurrence at 6 months in patients with symptomatic PE with or without symptomatic DVT.

### 2) Study design

Patients with confirmed symptomatic PE with or without acute symptomatic DVT are eligible for this trial. Treatment with a therapeutic dose of any LMWH or UFH or fondaparinux is allowed only within the 36 hours immediately preceding randomization. Randomization is performed as soon as the diagnosis of PE (and DVT if concomitant suspected symptomatic DVT) is confirmed.

Treatment duration is 3 months or 6 months of SSR126517E (or its placebo), or INR-adjusted warfarin (or its placebo), depending on baseline risk of DVT/PE recurrence, determined by the investigator prior to randomization.

A blinded central and independent adjudication committee (CIAC) assesses PE (and DVT when applicable), all episodes of locally confirmed or doubtful PE/DVT recurrences, clinically relevant bleedings and deaths. Adjudication results are the basis for the final analyses.

Patients in the 3-month stratum have an additional 13-week observational period after cessation of study treatment. Patients in the 6-month stratum have a 13-week up to 26-week observational period.

### 3) Study population

### 3-1: Inclusion criteria

The following patients were included in the study:
Patients with confirmed acute symptomatic PE, with or without symptomatic DVT of the lower limbs.

### 3-2 : Exclusion criteria

The following patients were excluded from enrollment in the study:
- Criteria related to study methodology :
   1. Legal lower age limitations (country specific).
   2. Women with childbearing potential without proper contraceptive measures.
   3. Written informed consent not obtained.
   4. Other indication than current episode of PE for anticoagulation.
   5. Thrombectomy, insertion of a caval filter, or use of a fibrinolytic agent to treat the current episode of PE.
   6. Treatment with a therapeutic dose of any LMWH or UFH or fondaparinux for more than 36 hours immediately preceding randomization.
   7. Life expectancy <6 months.
   8. Participation, within the prior 30 days, in another pharmacotherapeutic study with non-registered Investigational product.
- Criteria related to warfarin or enoxaparin :
   9. Pregnancy.
   10. Threatened abortion.
   11. Active major bleeding.
   12. Hemorrhagic tendencies or blood dyscrasias.
   13. Recent or contemplated surgery of central nervous system, eye, traumatic surgery resulting in large open surfaces.
   14. Spinal puncture and other diagnostic or therapeutic procedures with potential for uncontrollable bleeding.
   15. Malignant hypertension.
   16. Unsupervised patients with senility, alcoholism, or psychosis, or other lack of patient cooperation.
   17. Known hypersensitivity to warfarin or to any other component of this product.
   18. Patients with hypersensitivity to enoxaparin sodium, heparin, or pork products.
   19. Any other contraindication listed in the local warfarin or enoxaparin labelling.
- Criteria related to SSR126517E :
   20. Breast-feeding.
   21. Creatinine clearance <10 mL/min or end-stage renal failures
   22. Hypersensitivity to idraparinux or SSR126517E.
- Criteria related to avidin :
   23. Known allergy to avidin or egg proteins.

### 4) Investigational products

### 4-1 : Formulations

- SSR126517E : a sterile, pyrogen-free, isotonic solution with sodium chloride and water for injection s.c. Each milliliter of the solution contains 6 mg SSR126517E.
   SSR126517E is provided in pre-filled syringes:
   - containing 0.5 mL of this solution for the 3.0 mg dosage for patients without severe renal insufficiency (SRI) (creatinine clearance > 30 mL/min) and the first injection of patients with SRI (creatinine clearance ≤ 30 mL/min);
   - containing 0.3 mL (1.8 mg) of this solution for patients with SRI (injections following the first 3.0 mg injection).

   - Placebo of SSR126517E is a sterile, pyrogen-free, isotonic solution with sodium chloride and water for injection (s.c.). Placebo of SSR126517E is provided in pre-filled syringes:
      - containing 0.5 mL of this solution for patients without SRI;
      - containing 0.3 mL of this solution for patients with SRI.
   - Warfarin : tablets of 5 mg and 1 mg strengths, masked in capsules, and corresponding placebo of warfarin.
   - SSR29261 (avidin) : sterile, pyrogen-free, lyophilized powder, supplied in stoppered, clear, glass vials, containing 55 mg of avidin, to be reconstituted prior to administration with 5.5 mL of water for injection or of physiologic saline, thus resulting in a solution for i.v. injection at 10 mg/mL. 10 mL are to be diluted up to 110 mL, to enable i.v. infusion of 100 mg.
   - Placebo of SSR29261 (avidin) : supplied in stoppered, clear, glass vials, containing 55 mg of sterile, pyrogen-free, lyophilized excipient powder, with the same appearance as avidin powder, and to be reconstituted and administered the same way as described above for avidin.
   - Enoxaparin : locally marketed, pre-filled syringes as locally registered for b.i.d. treatment of venous thrombo-embolic disease (1.0 mg/kg every 12 hours).

### 4-2 : Routes of administration

- Enoxaparin, SSR126517E and placebo of SSR126517E: subcutaneous (s.c).
- Warfarin and placebo of warfarin: per os.
- SSR29261 or placebo of SSR29261: intravenous (i.v.) infusion over 30 minutes.

### 5) Dose regimen

Treatment with a therapeutic dose of any LMWH or UFH or fondaparinux is allowed only within the 36 hours immediately preceding randomization.

Post-randomization subcutaneous injections of enoxaparin is given at the dose of 1.0 mg/kg b.i.d. Hospitalization at this initial phase of the treatment should take place whenever this is specified in the local enoxaparin labelling for treatment of PE. Platelet count is regularly monitored during treatment with enoxaparin.

Minimal duration of treatment with enoxaparin is 5 days (including when applicable enoxaparin given before randomization for treatment of VTE at the dose of 1.0 mg/kg b.i.d.). Enoxaparin, overlapping with warfarin (or placebo of warfarin) for an advised duration of 4 to 5 days, is stopped after 2 consecutive values of centralized INR (true values in warfarin group, mock values in SSR126517E group) is ≥ 2.0 at least 24 hours apart.

Warfarin (or its placebo) should be started within 24 hours after randomization and given for a period of 3 or 6 months depending on baseline risk factors for VTE recurrence. Warfarin (or its placebo) dosage is adjusted to maintain the (true or mock) centralized INR within the therapeutic range (target 2.5, range 2.0-3.0). The INR should be checked at least once every month. In the case when the usual threshold of INR value that triggers an administration of Vitamin K is reached, this can be done without breaking the blind (i) only if the patient has no associated bleeding that absolutely requires the knowledge of the anticoagulant received, and (ii) provided vitamin K is administered orally, or, if not possible orally, by subcutaneous route only. The blind should be broken if the patient is symptomatic and his/her condition absolutely requires the knowledge of the anticoagulant received; or if vitamin K must be administered by intravenous route.

Injections of SSR126517E (or its placebo) is started 12h after the last post-randomization enoxaparin administration. SSR126517E (or its placebo) is administered s.c. once-weekly for a period of 3 or 6 months. All patients receive a first 0.5 ml injection (3.0 mg in the SSR126517E group). The following injections are also 0.5 ml, except in patients with severe renal insufficiency (defined as creatinine clearance < 30 mL/min), who decrease to a dosage of 0.3 ml (1.8 mg in the SSR126517E group).

One i.v. open-label infusion of 110 ml of the avidin solution (i.e. 100 mg of extractive avidin) may be administered in SSR126517E patients, after breaking the blind, in case of severe bleeding, or emergency invasive procedure with the potential of uncontrolled bleeding, or overdosage, whenever possible and appropriate.

In case of planned invasive procedure with the potential of uncontrolled bleeding, one i.v. infusion of 110 ml of avidin or of a placebo of avidin can be administered, without the need for breaking the blind, before the procedure, and after a 4-day interruption of study capsules.

Avidin (open label or double blind) can be administered during treatment with weekly injections and within 2 weeks following the last weekly injection.

### 6) Primary outcomes and main secondary outcomes

### 6-1: Efficacy

The primary efficacy outcome is symptomatic recurrent PE/DVT (fatal or not), as validated by the CIAC, within 99 days for patients from both strata (3- and 6-month treatments).

A secondary efficacy outcome for patients in the 6-month stratum is symptomatic recurrent PE/DVT, as validated by the CIAC, within 190 days.

There are 2 additional efficacy outcomes:
- the time to first symptomatic recurrent PE/DVT (fatal or non fatal) within the patient's treatment period, i.e. a combined analysis of the 3-and 6-month strata patients ;
- the separate components of the efficacy outcomes within 99 days (all patients) and within 190 days (6-month stratum patients only).

The definitions to confirm a suspected symptomatic episode of recurrent PE/DVT are as follows :
1 - PE: one of the following findings:
   - A new intraluminal filling defect in (sub)segmental or more proximal branches on spiral CT scan.
   - A new intraluminal filling defect or an extension of an existing defect or a new sudden cut-off of vessels more than 2.5 mm in diameter on the pulmonary angiogram.
   - A new perfusion defect of at least 75 % of a segment without a matching ventilation defect (high-probability) on ventilation/perfusion lung scintigraphy (VPLS).
   - Inconclusive spiral CT, pulmonary angiography or lung scintigraphy with demonstration of DVT in the lower extremities by compression ultrasound or venography.
   *OR*
2 - DVT of the lower limbs: one of the following findings:
   If there were no previous DVT investigations:
      - abnormal compression ultrasound (CUS),
      - an intraluminal-filling defect on venography.
   If there was a DVT investigation at screening:
      - abnormal CUS where compression had been normal or, if non-compressible during screening, a substantial increase (4 mm or more) in diameter of the thrombus during full compression,
      - an extension of an intraluminal filling defect, or a new intraluminal filling defect or an extension of non-visualization of veins in the presence of a sudden cut-off on venography.
   *OR*
3 - Fatal PE objectively documented.
   *OR*
4 - Death which cannot be attributed to a documented cause and for which DVT/PE cannot be ruled out.

The diagnosis of recurrence of PE is done exclusively using perfusion/ventilation lung scan, spiral computed tomography (CT) scan, pulmonary angiography, alone or in combination, compression ultrasonography (CUS) in case of inconclusive lung scan or spiral CT scan.

The diagnosis of DVT is done preferably by a bilateral venography or a CUS (including measurements of the diameters of the iliac, common femoral, popliteal and trifurcation veins, before and after compression) of the lower limbs. In case of associated acute symptomatic DVT at randomization, or exploration of the deep veins of the legs to ascertain the diagnosis of the initial PE, the same type of examination as the one used at randomization is performed to confirm or rule out the DVT recurrence.

### 6-2 : Safety

The principal safety outcome is any clinically relevant bleeding as classified by the CIAC (i.e. major bleeding and other clinically relevant non-major bleeding).

### 7) Assessment schedule

### 7-1 : Screening phase

Screening phase takes place within 48 hours before randomization.

Patients with acute symptomatic PE are potentially eligible for the study only if the following laboratory examinations are performed before randomization: perfusion/ventilation lung scan, Spiral CT scan, pulmonary angiography, and, if necessary, bilateral venography or Compression Ultrasound of the lower limbs with measurement of deep veins diameters.

The diagnosis of PE is based on either one of the following:
- an intraluminal filling defect in (sub)segmental or more proximal branches on spiral CT scan,
- an intraluminal filling defect or a sudden cut-off of vessels more than 2.5 mm in diameter on the pulmonary angiogram,
- a perfusion defect of at least 75% of a segment without a matching ventilation defect (high-probability) on VPLS,
- inconclusive spiral CT, pulmonary angiography or lung scintigraphy with demonstration of DVT in the lower extremities by compression ultrasound or venography.

If an associated acute symptomatic DVT is suspected, the diagnosis shall be confirmed before randomization by a bilateral venography or a CUS including measurements of the diameters of the iliac, common femoral, popliteal and trifurcation veins, before and after compression of the lower limbs.

The diagnosis of DVT is based on either one of the following:
- a non-compressible vein in the trifurcation of the calf veins and/or more proximal veins CUS,
- an intraluminal filling defect in the trifurcation of the calf veins and/or more proximal veins on venography.

### 7-2 : After randomization

After randomization, there are contacts (hospital/clinic visits or telephone calls) at beginning of week 2 (D8±1-day window), week 3 (D15±1-day window), week 4 (D22±1-day window). A visit is mandatory at week 7 (D43±3-day window) for re-supply of warfarin or its placebo. A hospital/clinic visit is mandatory just after the first injection of SSR126517E or its placebo and at D15±1-day window for SRI patients.

At 3 months (D99 [+7-day window]) and 6 months (D190 [+7-day window]) from randomization, all patients have a hospital/clinic visit.

Platelet count, ASAT, ALAT, creatininemia, and pregnancy test are measured at the end of the study treatment in all patients.
- Platelet count is regularly monitored during treatment with enoxaparin.
- Creatinemia is systematically checked in case of renal disease or extra-renal condition likely to affect renal function.

ALAT and total bilirubin (and, if total bilirubin > x 2ULN, conjugated bilirubin) are measured:
- systematically at D99 [+7-day window] in the 3-month stratum; and at D99 [+7-day window] and at D190 [+7-day window] in the 6-month stratum, or within the 2 weeks following permanent study drug discontinuation, whichever comes first;
- whenever there is a clinical suspicion of liver damage.

Permanent study drug discontinuation takes place when a concomitant increase in x 3 ULN of ALAT and in x 2 ULN of total bilirubin (with predominant conjugated bilirubin) is observed, and confirmed on a second test performed within 48 hours. These tests are re-checked at the latest 7 days later, and followed up to the return to normal or stable condition.

VTE recurrences are assessed until the end of study by the laboratory examination listed above.

Open-label avidin (SSR29261) is available to reverse anticoagulation whenever deemed necessary: in case of life-threatening bleeding, emergency invasive procedures with the potential of uncontrolled bleeding, or overdosage. Double blind administration of avidin or its placebo takes place before a planned invasive procedure with the potential of uncontrolled bleeding, and after a 4 day interruption of study capsules. It could be repeated in the same circumstances if administration of idrabiotaparinux/placebo of idrabiotaparinux was resumed after a previous double-blind administration of avidin or its placebo.

### 8) Statistical considerations

### - Population analyzed

Efficacy analyses are performed on the « All-randomized » population (patients with a patient number and an allocated treatment number recorded in the IVRS database).

The primary efficacy outcome is also analysed on the « Per Protocol » population (secondary efficacy analysis).

Bleeding analyses are also performed on the « All-randomized » population. Other safety analyses are performed on the « All-treated » population.

### - Sample size

Assuming a 2.4% incidence of VTE in the standard therapy group at 3 months and a pre-specifed non-inferiority margin of 2.0 for the odds ratio, a sample size of 1600 patients per group was necessary to show that idrabiotaparinux was at least as effective than warfarin with 85% power and a one-sided type I error of 0.025.

### - Efficacy analysis

If the upper limit of the 95% confidence interval for the SSR126517E to warfarin odds ratio, stratified on intended treatment duration (Mantel-Haenszel chi-square analysis), for the primary efficacy outcome (VTE recurrence within 99 days) is less than 2.0, idrabiotaparinux is considered at least as effective as warfarin.

The secondary efficacy outcome (VTE recurrence within 190 days) for patients in the 6-month stratum is analyzed using the same method and applying the same non-inferiority margin.

Time to first symptomatic recurrent PE/DVT within the patient's study treatment period for all patients is analyzed using Cox's proportional hazards model, stratified on intended treatment duration.

### - Safety Analysis

Rates of clinically relevant bleedings are calculated in each treatment group at 3 months (99 days) and 6 months (190 days) and compared between treatment groups using a Mantel-Haenszel chi-square stratified on intended treatment duration. Cumulative incidences of clinically relevant bleedings during the combined 3-month and 6-month period and up to the end of study are described by treatment group using the Kaplan Meier method.

### 9) Duration of study period (per patient)

The duration of treatment, pre-specified by the investigator, is 3 or 6 months (depending on assessed risk for recurrent PE/DVT), followed by an additional observational period of 13 weeks (3-month stratum) or 13 to 26 weeks (6-month stratum).

### 10) Results

A total of 3202 patients were randomized, 1599 in the idrabiotaparinux group and 1603 in the warfarin group.

The demographic characteristics of randomized patients were similar in both treatment groups.

79% of patients were included in the 6-month stratum in the idrabiotaparinux and warfarin groups.

### 10-1: Efficacy results

### - Primary analysis

Idrabiotaparinux was at least as effective as warfarin in reducing the risk of symptomatic recurrent PE/DVT up to Day 99 (p < 0.0001 for testing non-inferiority) in the all randomized population. The upper bound of the 95% Cl was 1.25, lower than the prespecified 2.0 non-inferiority margin.

**Table 1 - Symptomatic recurrent PE/DVT (fatal or not) within 99 days from randomization - Randomized population**

| | Idrabiotaparinux (N=1599) | Warfarin (N=1603) |
|---|---|---|
| Symptomatic recurrent PE/DVT (fatal or not) within 99 days from randomization | | |
| Patients with event [n (%)] | 34(2.1%) | 43 (2.7%) |
| 95% Cl | (1.5% to 3.0%) | (1.9% to 3.6%) |
| Idrabiotaparinux vs. Warfarin | | |
| Odds ratio | 0.79 | - |
| 95% Cl | (0.50 to 1.25) | - |
| p-value^{a} | <0.0001 | - |

| | | |
|---|---|---|
| Note: Odds ratio, 95% Cl and p-value determined from a Mantel-Haenszel test, stratified on intended treatment duration (3 or 6-month stratum) ^{a} p-value for testing the non-inferiority hypothesis (non-inferiority margin: 2.0) at a significance level of 0.025 | | |

### - Separate components of the symptomatic recurrent PE/DVT within 3 months

The incidence of symptomatic recurrent fatal or non-fatal PE up to Day 99 was similar between the two treatment groups, while fewer patients experienced DVT in the idrabiotaparinux group (0.3%) than in the warfarin group (1.1 %).

**Table 2 - Separate components of symptomatic recurrent PE/DVT (fatal or not) within 99 days from randomization - Randomized population**

| | Idrabiotaparinux (N=1599) | Warfarin (N=1603) |
|---|---|---|
| Fatal PE^{a} | | |
| Patients with event [n (%)] | 16 (1.0%) | 16 (1.0%) |
| 95% Cl | (0.6% to 1.6%) | (0.6% to 1.6%) |
| Non-fatal PE | | |
| Patients with event [n (%)] | 13 (0.8%) | 9 (0.6%) |
| 95% Cl | (0.4% to 1.4%) | (0.3% to 1.1%) |
| DVT only | | |
| Patients with event [n (%)] | 5 (0.3%) | 18 (1.1%) |
| 95% Cl | (0.1 % to 0.7%) | (0.7% to 1.8%) |

| | | |
|---|---|---|
| Note: a patient is counted only once, in the worst category ^{a} or death which could not be attributed to a documented cause or for which PE could not be ruled out | | |

### - Symptomatic recurrent PE/DVT within 6 months

Idrabiotaparinux was also at least as effective as warfarin in reducing the risk of symptomatic recurrent PE/DVT up to Day 190 (p = 0.0001 for testing non-inferiority) in patients randomized in the 6-month stratum. The upper bound of the 95% Cl was 1.31, lower than the prespecified 2.0 non-inferiority margin.

**Table 3 - Symptomatic recurrent PE/DVT (fatal or not) within 190 days from randomization - Randomized population in the 6-month stratum**

| | Idrabiotaparinux (N=1269) | Warfarin (N=1267) |
|---|---|---|
| Symptomatic recurrent PE/DVT (fatal or not) within 190 days from randomization | | |
| Patients with event [n (%)] | 29 (2.3%) | 36 (2.8%) |
| 95% Cl | (1.5% to 3.3%) | (2.0% to 3.9%) |
| Idrabiotaparinux vs. Warfarin | | |
| Odds ratio | 0.80 | - |
| 95% Cl | (0.49 to 1.31) | - |
| p-value^{a} | 0.0001 | - |

| | | |
|---|---|---|
| ^{a} p-value for testing the non-inferiority hypothesis (non-inferiority margin: 2.0) at a significance level of 0.025 | | |

Within patients randomized in the 6-month stratum, the incidence of symptomatic recurrent fatal or non-fatal PE up to Day 190 was similar between the two treatment groups, while fewer patients experienced DVT in the idrabiotaparinux group (0.6%) than in the warfarin group (1.3%).

**Table 4 - Separate components of symptomatic recurrent PE/DVT (fatal or not) within 190 days from randomization - Randomized population in the 6-month stratum**

| | Idrabiotaparinux (N=1269) | Warfarin (N=1267) |
|---|---|---|
| Fatal PE^{a} | | |
| Patients with event [n (%)] | 13 (1.0%) | 9 (0.7%) |
| 95% Cl | (0.5% to 1.7%) | (0.3% to 1.3%) |
| Non-fatal PE | | |
| Patients with event [n (%)] | 9 (0.7%) | 10 (0.8%) |
| 95% Cl | (0.3% to 1.3%) | (0.4% to 1.4%) |
| DVT only | | |
| Patients with event [n (%)] | 7 (0.6%) | 17 (1.3%) |
| 95% Cl | (0.2% to 1.1%) | (0.8% to 2.1 %) |

| | | |
|---|---|---|
| Note: a patient is counted only once, in the worst category ^{a} or death which could not be attributed to a documented cause or for which PE could not be ruled out | | |

### - Symptomatic recurrent PE/DVT in the combined 3-month and 6-month period

Combining the 3-month and 6-month periods and taking into account censored data using a time-to-event analysis, the cumulative incidence of symptomatic recurrent PE/DVT at Day 99 were 2.2% in the idrabiotaparinux group and 2.7% in the warfarin group. At Day 190, these cumulative incidences were 2.6% and 3.4% respectively. The hazard ratio was 0.77 (95% Cl: 0.51 to 1.17).

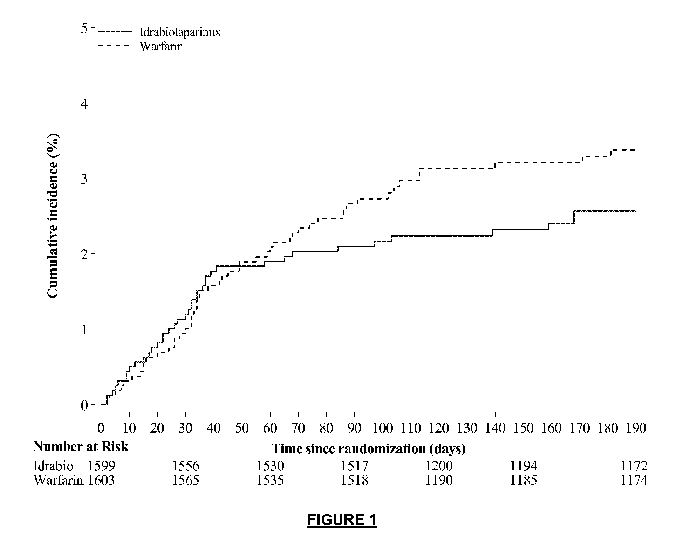

The Kaplan-Meier cumulative incidence curve (Figure 1) showed that the incidence of PE/DVT was similar between the two groups up to approximately Day 50, and then started to diverge with less events in the idrabiotaparinux group.

### - Symptomatic recurrent PE/DVT up to the end of the study

Taking into account all events that occurred either during the treatment period or during the 3 to 6-month post-treatment follow-up period, the cumulative incidence rates of symptomatic recurrent PE/DVT at Day 360 were 3.1% in the idrabiotaparinux group and 7.2% in the warfarin group. The hazard ratio was 0.49 with 95% Cl (0.35 to 0.70), demonstrating superiority of efficacy at 12 months for idrabiotaparinux compared to warfarin.

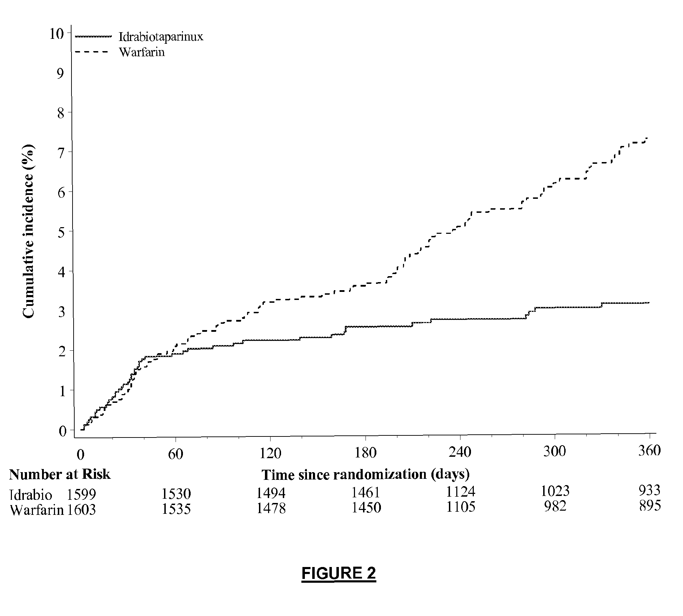

The Kaplan-Meier cumulative incidence curve (Figure 2) showed that after Day 190, the incidence of PE/DVT tended to increase in the warfarin group, while it remained stable in the idrabiotaparinux group.

The incidence of fatal PE up to the end of study was similar between the two treatment groups, while fewer patients experienced non-fatal PE (1.0% versus 2.6%) or DVT (0.6% versus 1.9%) in the idrabiotaparinux group.

**Table 5 - Separate components of symptomatic recurrent PE/DVT (fatal or not) up to the end of study - Randomized population**

| | Idrabiotaparinux (N=1599) | Warfarin (N=1603) |
|---|---|---|
| Fatal PE^{a} | | |
| Patients with event [n (%)] | 22 (1.4%) | 25 (1.6%) |
| 95% Cl | (0.9% to 2.1 %) | (1.0% to 2.3%) |
| Non-fatal PE | | |
| Patients with event [n (%)] | 16 (1.0%) | 41 (2.6%) |
| 95% Cl | (0.6% to 1.6%) | (1.8% to 3.5%) |
| DVT only | | |
| Patients with event [n (%)] | 10 (0.6%) | 31 (1.9%) |
| 95% Cl | (0.3% to 1.1%) | (1.3% to 2.7%) |

| | | |
|---|---|---|
| Note: a patient is counted only once, in the worst category ^{a} or death which could not be attributed to a documented cause or for which PE could not be ruled out | | |

### - Symptomatic recurrent PE/DVT up to the end of the study according to switch to VKA

Further efficacy analyses were carried out on the randomized population, amongst patients having or not having being treated with warfarin after their initial 3-month (D99) or 6-month (D190) treatment with idrabiotaparinux.

As shown in table 6, patients not receiving warfarin beyond their idrabiotaparinux treatment have a similar incidence of recurrent thromboembolic events at 12 months (end of the study) than patients treated with warfarin beyond the initial treatment with idrabiotaparinux, demonstrating a protective effect of idrabiotaparinux after discontinuation of idrabiotaparinux treatment.

Table 7 shows the incidence of thromboembolic events in the period after idrabiotaparinux discontinuation (from D100/D191 up to the end of the study).

**Table 6 - Symptomatic recurrent PE/DVT (fatal or not) from randomization to end of study, according to switch to VKA beyond D99/D190 - Randomized population**

| | Idrabiotaparinux (N=1599) | Warfarin (N=1603) |
|---|---|---|
| All patients | | |
| Number of patients | 1599 | 1606 |
| Patients with event [n (%)] | 48 (3.0%) | 97(6.1%) |
| Patients with VKA beyond D99/D190 | | |
| Number of patients | 387 | 484 |
| Patients with event [n (%)] | 12(3.1%) | 45 (9.3%) |
| Patients with no VKA beyond D99/D190 | | |
| Number of patients | 1212 | 1119 |
| Patients with event [n (%)] | 36 (3.0%) | 52 (4.6%) |

**Table 7 - Symptomatic recurrent PE/DVT (fatal or not) from D100/D191 to end of study, according to switch to VKA beyond D99/D190 - Randomized population**

| | Idrabiotaparinux (N=1599) | Warfarin (N=1603) |
|---|---|---|
| All patients | | |
| Number of patients | 1478 | 1495 |
| Patients with event [n (%)] | 9 (0.6%) | 47(3.1%) |
| Patients with VKA beyond D99/D190 | | |
| Number of patients | 385 | 482 |
| Patients with event [n (%)] | 4 (1.0%) | 32 (6.6%) |
| Patients with no VKA beyond D99/D190 | | |
| Number of patients | 1093 | 1013 |
| Patients with event [n (%)] | 5 (0.5%) | 15 (1.5%) |

| | | |
|---|---|---|
| Note : Patients with no assessment of recurrent PE/DVT after D100/D191 are excluded from the analysis. | | |

Table 8 shows the results amongst the patients initially treated with idrabiotaparinux for 6 months, i.e. most (about 80%) of the randomized patients.

**Table 8 - Symptomatic recurrent PE/DVT (fatal or not) from randomization to end of study, according to switch to VKA beyond D190 - Randomized population in the 6-month stratum**

| | Idrabiotaparinux (N=1269) | Warfarin (N=1267) |
|---|---|---|
| All patients | | |
| Number of patients | 1269 | 1267 |
| Patients with event [n (%)] | 37 (2.9%) | 76 (6.0%) |
| Patients with VKA beyond D190 | | |
| Number of patients | 317 | 407 |
| Patients with event [n (%)] | 10 (3.2%) | 37(9.1%) |
| Patients with no VKA beyond D190 | | |
| Number of patients | 952 | 860 |
| Patients with event [n (%)] | 27 (2.8%) | 39 (4.5%) |

### 10-2: Safety results

### - Bleedings within 3 months

There was a statistically significant reduction in incidence of clinically relevant bleeding (i.e. major bleeding or clinically relevant non-major bleeding) up to Day 99 in the idrabiotaparinux group. In the idrabiotaparinux group, the incidence of clinically relevant bleeding was 4.5% versus 6.6% in the warfarin group (p=0.0098 for testing superiority).

**Table 9 - Clinically relevant bleeding within 99 days from randomization - Randomized population**

| | Idrabiotaparinux (N=1599) | Warfarin (N=1603) |
|---|---|---|
| Any clinically relevant bleeding | | |
| Patients with event [n (%)] | 72 (4.5%) | 106 (6.6%) |
| 95% Cl | (3.5% to 5.6%) | (5.4% to 7.9%) |
| Idrabiotaparinux vs. Warfarin | | |
| Odds ratio | 0.67 | - |
| 95% Cl | (0.49 to 0.91) | - |
| p-value^{a} | 0.0098 | - |

| | | |
|---|---|---|
| Note: Odds ratio, 95% Cl and p-value determined from a Mantel-Haenszel test, stratified on intended treatment duration (3 or 6-month stratum) ^{a} p-value for testing the superiority hypothesis at a significance level of 0.05 (two-sided) | | |

Fewer patients experienced a major bleeding up to Day 99 in the idrabiotaparinux group (1.3%) than in the warfarin group (2.4%), with an odds-ratio 0.51 (95% Cl from 0.30 to 0.88). The incidence of intracranial hemorrhages and fatal bleedings were similar between the two groups.

**Table 10 - Major bleeding within 99 days from randomization - Randomized population**

| | Idrabiotaparinux (N=1599) | Warfarin (N=1603) | Idrabiotaparinux to Warfarin odds ratio (95% Cl) |
|---|---|---|---|
| Major bleeding | | | |
| Patients with event [n (%)] | 20 (1.3%) | 39 (2.4%) | 0.51 (0.30 to 0.88) |
| 95% Cl | (0.8% to 1.9%) | (1.7% to 3.3%) | |
| Intracranial haemorrhage (fatal or not) Patients with event [n (%)] | 4 (0.3%) | 7 (0.4%) | 0.57 (0.17 to 1.96) |
| 95% Cl | (0.1 % to 0.6%) | (0.2% to 0.9%) | |
| Fatal bleeding Patients with event [n (%)] | 3 (0.2%) | 6 (0.4%) | 0.50 (0.13 to 2.01) |
| 95% Cl | (0.0% to 0.5%) | (0.1 % to 0.8%) | |

| | | | |
|---|---|---|---|
| Note: Odds ratio, 95% Cl and p-value determined from a Mantel-Haenszel test, stratified on intended treatment duration (3 or 6-month stratum) | | | |

### - Bleedings within 6 months

Within patients randomized in the 6-month stratum, there were less clinically relevant bleedings up to Day 190 in the idrabiotaparinux group compared to the warfarin group: 6.6% versus 8.1%. This difference was not statistically significant (p=0.1675) but showed a trend of superiority for idrabiotaparinux.

**Table 11 - Clinically relevant bleeding within 190 days from randomization - Randomized population in the 6-month stratum**

| | Idrabiotaparinux (N=1269) | Warfarin (N=1267) |
|---|---|---|
| Any clinically relevant bleeding | | |
| Patients with event [n (%)] | 84 (6.6%) | 102 (8.1 %) |
| 95% Cl | (5.3% to 8.1 %) | (6.6% to 9.7%) |
| Idrabiotaparinux vs. Warfarin | | |
| Odds ratio | 0.81 | - |
| 95% Cl | (0.60 to 1.09) | - |
| p-value^{a} | 0.1675 | - |

| | | |
|---|---|---|
| ^{a} p-value for testing the superiority hypothesis at a significance level of 0.05 (two-sided) | | |

Fewer patients experienced a major bleeding up to Day 190 in the idrabiotaparinux group (1.9%) than in the warfarin group (2.8%). The incidence of intracranial hemorrhages and fatal bleedings were similar between the two groups.

**Table 12 - Major bleeding within 190 days from randomization - Randomized population in the 6-month stratum**

| | Idrabiotaparinux (N=1269) | Warfarin (N=1267) | Idrabiotaparinux to Warfarin odds ratio (95% Cl) |
|---|---|---|---|
| Major bleeding | | | |
| Patients with event [n (%)] | 24 (1.9%) | 36 (2.8%) | 0.66 (0.39 to 1.11) |
| 95% Cl | (1.2% to 2.8%) | (2.0% to 3.9%) | |
| Intracranial haemorrhage (fatal or not) | | | |
| Patients with event [n (%)] | 8 (0.6%) | 6 (0.5%) | 1.33 (0.46 to 3.85) |
| 95% Cl | (0.3% to 1.2%) | (0.2% to 1.0%) | |
| Fatal bleeding | | | |
| Patients with event [n (%)] | 7 (0.6%) | 5 (0.4%) | 1.40 (0.44 to 4.42) |
| 95% Cl | (0.2% to 1.1%) | (0.1 % to 0.9%) | |

### - Bleedings in the combined 3-month and 6-month period

Combining the 3-month and 6-month periods and taking into account censored data using a time-to-event analysis, the cumulative incidence of clinically relevant bleeding at Day 99 were 4.6% in the idrabiotaparinux group and 6.7% in the warfarin group. At Day 190, these cumulative incidences were 6.6% and 9.1% respectively. The hazard ratio was 0.70 (95% Cl: 0.54 to 0.91).

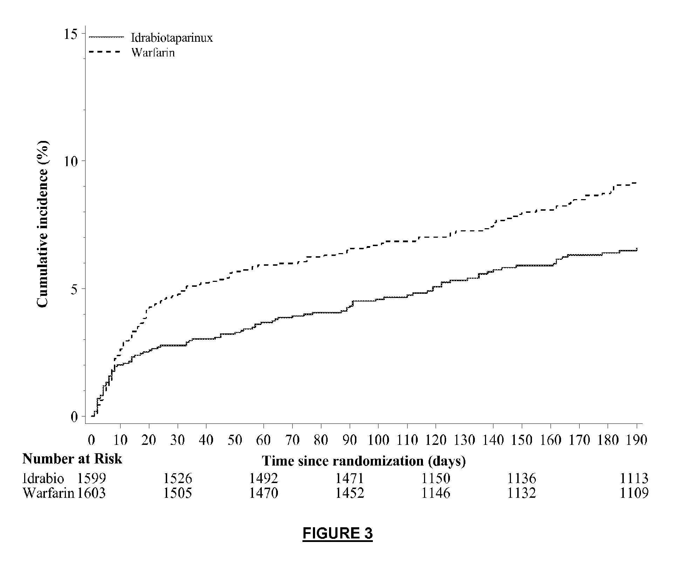

The Kaplan-Meier cumulative incidence curve (Figure 3) showed that the incidence of bleedings was similar between the two groups up to Day 8, and then started to diverge with less events in the idrabiotaparinux group.

### - Bleedings up to the end of study

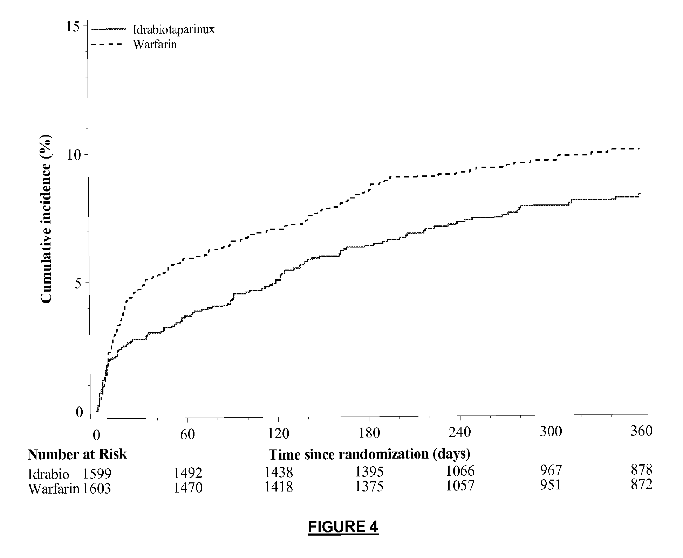

Taking into account all bleedings that occurred either during the treatment period or during the 3 to 6-month post-treatment follow-up period, the cumulative incidence rates of clinically relevant bleeding at Day 360 were 8.3% in the idrabiotaparinux group and 10.0% in the warfarin group (see Figure 4). The hazard ratio was 0.79 with 95% Cl (0.62 to 1.00).

### - Deaths

Overall, the number of patients who died on study was similar in both treatment groups (5.9% in the idrabiotaparinux group versus 5.3% in the warfarin group).

### 10-3: Benefit-risk assessment

Tables 13 and 14 show the incidence of pulmonary embolism, deep venous thrombosis or major bleedings (composite endpoint) in the randomized population in the treatment phase (3-month or 6-month treatment with idrabiotaparinux) and up to the end of the study (assessment at 12 months). These results demonstrate the improved benefit-risk ratio of idrabiotaparinux versus warfarin, within the treatment duration as well as beyond (up to 12 months).

Table 13 - PE, DVT or major bleeding in the combined 3-month and 6-month period - Randomized population

| | Idrabiotaparinux (N=1559) | Warfarin (N=1603) |
|---|---|---|
| PE, DVT or major bleeding in the combined 3-month and 6-month period | | |
| Patients with event [n (%)] | 65(4.1%) | 98 (6.1 %) |
| 95% Cl | (3.2% to 5.2%) | (5.0% to 7.4%) |
| Idrabiotaparinux vs. Warfarin | | |
| Odds ratio | 0.66 | - |
| 95% Cl | (0.48 to 0.91) | - |
| p-value | 0.0098 | - |

| | | |
|---|---|---|
| Note: Hazard ratio, 95% Cl and p-value determined using a Cox proportional hazards model, stratified on intended treatment duration (3 or 6-month stratum). | | |

**Table 14 - PE, DVT or major bleeding up to the end of study - Randomized population**

| | Idrabiotaparinux (N=1559) | Warfarin (N=1603) |
|---|---|---|
| PE, DVT or major bleeding up to the end of study | | |
| Patients with event [n (%)] | 82(5.1%) | 150 (9.4%) |
| 95% Cl | (4.1% to 6.3%) | (8.0% to 10.9%) |
| Idrabiotaparinux vs. Warfarin | | |
| Odds ratio | 0.54 | - |
| 95% Cl | (0.41 to 0.71) | - |
| p-value | < 0.0001 | - |

| | | |
|---|---|---|
| Note: Hazard ratio, 95% Cl and p-value determined using a Cox proportional hazards model, stratified on intended treatment duration (3 or 6-month stratum). | | |

### 11) Conclusion

In this trial idrabiotaparinux has been demonstrated as non-inferior to warfarin for the treatment of PE and prevention of recurrences of venous thromboembolic events in patients with PE with or without DVT. In addition, idrabiotaparinux has been demonstrated to be superior to warfarin for the safety endpoint (in particular clinically relevant bleedings).

Idrabiotaparinux has a better and significant net clinical benefit at 3 and 6 months after start of the treament, with a protective effect up to 12 months, without increase in the risk of bleeding.

## Claims

1. Idrabiotaparinux for use in the treatment of pulmonary embolism in patients with or without deep venous thrombosis and for the secondary prevention of venous thromboembolic events in said patients, wherein the efficacy and safety of said uses are clinically proven by a phase III clinical trial.

2. Idrabiotaparinux according to claim 1, wherein said clinical trial enrolled 3202 patients.

3. Idrabiotaparinux according to claim 1 or claim 2, wherein said clinical trial included patients with confirmed acute symptomatic pulmonary embolism, with or without deep venous thrombosis of the lower limbs.

4. Idrabiotaparinux according any of claims 1 to 3, wherein idrabiotaparinux is administered for 3 or for 6 months.

5. Idrabiotaparinux according to any of claims 1 to 4, wherein idrabiotaparinux is administered at a 3.0 mg dose once weekly for patients without severe renal insufficiency or at a 1.8 mg dose once weekly for patients with severe renal insufficiency.

6. Idrabiotaparinux according to any of claims 1 to 5, wherein the efficacy and safety of said uses are assessed compared to a vitamin K antagonist as standard antithrombotic treatment.

7. Idrabiotaparinux according to any of claims 1 to 6, wherein said venous thromboembolic events are selected from pulmonary embolism, fatal or not, and deep venous thrombosis.

8. Idrabiotaparinux according to any of claims 1 to 7, wherein idrabiotaparinux displays an improved safety in terms of bleedings, compared to a vitamin K antagonist as standard antithrombotic treatment.

9. Idrabiotaparinux according to claim 8, wherein idrabiotaparinux displays an improved safety in terms of major bleedings.

10. Idrabiotaparinux according to any of claims 1 to 9, wherein idrabiotaparinux displays an improved benefit-risk ratio compared to a vitamin K antagonist as standard antithrombotic treatment.

11. Idrabiotaparinux according to any of claims 6, 8 or 10, wherein said vitamin K antagonist is warfarin.

12. Idrabiotaparinux according to any of claims 1 to 11, wherein its efficacy in said uses and its improved benefit-risk ratio remain present after treatment discontinuation, up to 12 months after start of the treatment with idrabiotaparinux.

13. Idrabiotaparinux for use in the extended prevention of venous thromboembolic events for an additional 6-month period after discontinuation of an initial 6-month treatment with idrabiotaparinux, in patients with or without deep venous thrombosis.

14. An article of manufacture comprising:
- a packaging material,
- a compound chosen from idrabiotaparinux or a pharmaceutically acceptable salt thereof, and
- a label or package insert contained within said packaging material indicating that said compound is effective as an antithrombotic treatment for the treatment of pulmonary embolism in patients with or without deep venous thrombosis and for the secondary prevention of venous thromboembolic events in said patients.

15. An article of manufacture comprising:
- a packaging material,
- a compound chosen from idrabiotaparinux or a pharmaceutically acceptable salt thereof, and
- a label or package insert contained within said packaging material indicating that said compound is safe as an antithrombotic treatment for the treatment of pulmonary embolism in patients with or without deep venous thrombosis and for the secondary prevention of venous thromboembolic events in said patients.
